# EUROPEAN PATENT SPECIFICATION

(11) **EP 3 619 320 B1**
(45) Date of publication and mention of the grant of the patent: **11.09.2024**
(21) Application number: 18719616.7
(22) Date of filing: 02.05.2018
(51) Int. Cl.: C12Q 1/6883

(54) **PREDICTIVE RESPONSE BIOMARKERS TO GLUCOCORTICOSTEROIDS**
PRÄDIKTIVE BIOMARKER FÜR DIE REAKTION AUF GLUCOCORTICOSTEROIDE
BIOMARQUEURS DE RÉPONSE PRÉDICTIVE À DES GLUCOCORTICOSTÉROÏDES

(30) Priority: 03.05.2017 EP 17382246
(43) Date of publication of application: 11.03.2020
(73) Proprietor: Fundació Institut d'Investigació en Ciències de la Salut Germans Trias i Pujol, 08916 Badalona (ES); Centro de Investigación Biomédica en Red, 28029 Madrid (ES)
(72) Inventor: DOMÈNECH MORRAL, Eugeni, 08391 Tiana (ES); MANYÉ ALMERO, Josep, 08917 Badalona (ES); LORÉN MORENO, Violeta, 43800 Valls (ES); ENRIQUE NAVES, Juan, 08029 Barcelona (ES)
(74) Representative: ZBM Patents - Zea, Barlocci & Markvardsen
(86) International application number: PCT/EP2018/061219
(87) International publication number: WO 2018/202723

(56) References cited:
- WO-A1-2016/066288
- WO-A1-2016/092045
- WO-A2-2008/142567
- WO-A2-2012/174293
- SCHAEFER JEREMY S ET AL: "MicroRNA signatures differentiate Crohn's disease from ulcerative colitis", BMC IMMUNOLOGY, vol. 16, no. 1, 1 December 2015 (2015-12-01), XP093027816, Retrieved from the Internet <URL:https://bmcimmunol.biomedcentral.com/counter/pdf/10.1186/s12865-015-0069-0.pdf> DOI: 10.1186/s12865-015-0069-0
- THOMAS X LU ET AL: "MicroRNA signature in patients with eosinophilic esophagitis, reversibility with glucocorticoids, and assessment as disease biomarkers", JOURNAL OF ALLERGY AND CLINICAL IMMUNOLOGY, ELSEVIER, AMSTERDAM, NL, vol. 129, no. 4, 24 January 2012 (2012-01-24), pages 1064 - 1075.e9, XP028478871, ISSN: 0091-6749, [retrieved on 20120201], DOI: 10.1016/J.JACI.2012.01.060
- XU LING ET AL: "[Association of miRNAs expression profiles with prognosis and relapse in childhood acute lymphoblastic leukemia]", ZHONGHUA XUEYEXUE ZAZHI - CHINESE JOURNAL OF HEMATOLOGY, TIANJIN, CN, vol. 32, no. 3, 25 July 2012 (2012-07-25), pages 178 - 181, XP009161486, ISSN: 0253-2727
- NAVES J E ET AL: "DOP013 Pre-treatment differential microRNA expression profile in ulcerative colitis patients according to their response to corticosteroids", JOURNAL OF CROHN'S AND COLITIS, vol. 8, 1 May 2014 (2014-05-01) - 1 May 2014 (2014-05-01), XP028617626, ISSN: 1873-9946, DOI: 10.1016/S1873-9946(14)60038-6
- WILLIAMS ANDREW E ET AL: "MicroRNA Expression Profiling in Mild Asthmatic Human Airways and Effect of Corticosteroid Therapy", PLOS ONE, PUBLIC LIBRARY OF SCIENCE, US, vol. 4, no. 6, 1 June 2009 (2009-06-01), XP009134137, ISSN: 1932-6203, [retrieved on 20090612], DOI: 10.1371/JOURNAL.PONE.0005889

## Description

This application claims the benefit of European Patent Application EP17382246 filed May 3rd, 2017.

The present invention relates to methods for predicting the response to a corticosteroid-based therapy in a subject.

### BACKGROUND ART

Inflammatory diseases are a large group of disorders that underlie a vast variety of human diseases. The immune system is often involved with inflammatory disorders, demonstrated in both allergic reactions and some myopathies, with many immune system disorders resulting in abnormal inflammation. A large variety of proteins are involved in inflammation, and any one of them is open to a genetic mutation which impairs or otherwise dysregulates the normal function and expression of that protein.

Examples of disorders associated with inflammation include: acne vulgaris; asthma; autoimmune diseases; autoinflammatory diseases; celiac disease; chronic prostatitis; diverticulitis; glomerulonephritis; hidradenitis suppurativa; inflammatory bowel diseases; otitis; pelvic inflammatory disease; or rheumatoid arthritis, among others.

Inflammatory bowel disease (IBD) comprises Crohn's disease (CD) and ulcerative colitis (UC), both being known as causing long-term damage to the intestine. Recently, microscopic colitis, collagenous colitis and lymphocytic colitis have also been included in IBD. The combination of external antigens, impaired immune reactions and hereditary factors seem to contribute to the etiology of IBD, but the mechanisms initiating the intestinal inflammation remain unclear.

In CD, any part of the bowel can be affected, but most common is affection of the ileocaecal region (50%) and colon (30%). About one fourth of the patients also have concomitant perianal disease. Several short segments of the small bowel may be affected, often known as skip lesions. Histopathologically, the inflammation is transmural; dense infiltrations of lymphocytes and macrophages with granulomas are seen in up to 60 % of the patients. UC usually involves the rectum, and extends in a proximal direction, the extension varying between individual patients; the disease however always remains restricted to the colon. The term total colitis is used when the inflammation is also involving the right colonic flexure. Microscopically, mucosal inflammation is superficial and rich of lymphocytes and granulocytes. Ulcerations and crypt abscesses are common. Along with the symptoms from the prolonged inflammation such as diarrhoea (often containing blood), malnutrition, fever and pain, the patient is also at long- term risk of developing epithelial dysplasia and finally cancer in the intestine. About 15% of the IBD patients have indeterminate colitis and therefore a definite diagnosis of CD or UC cannot be set.

Even more important, among patients with fulminant colitis, there is often uncertainty regarding diagnosis. A correct diagnosis is important when deciding about immediate medical treatment and, in a longer time perspective, about the best surgical treatment.

Active UC is characterized by infiltration of activated granulocytes and monocytes/macrophages within the colonic mucosa. These infiltrating cells are major sources of inflammation-promoting cytokines. IBD patients may develop resistance towards medical treatment. Possible causes are changes in distribution of granulocytes, cytokines or functional characteristics of immune cells including the expression of steroid-receptors.

Today, the diagnosis and staging of IBD are based on a combination of clinical parameters, (albumin, SR, CRP, fever, number of stools) together with endoscopic criteria and histopathology.

The early accurate diagnosis of the disease is needed to efficiently treat the disease with the more appropriate therapy.

Corticosteroids are a class of steroid hormones that are produced in the adrenal cortex of vertebrates, as well as the synthetic analogues of these hormones. Two main classes of corticosteroids, glucocorticoids and mineralocorticoids, are involved in a wide range of physiologic processes, including stress response, immune response, and regulation of inflammation, carbohydrate metabolism, protein catabolism, blood electrolyte levels, and behavior.

Synthetic pharmaceutical drugs with corticosteroid-like effects are used in a variety of conditions to reduce inflammation and suppress the immune system, ranging from brain tumors to skin diseases. They are used to treat conditions such as: asthma; allergic rhinitis and hay fever urticarial (hives); atopic eczema; chronic obstructive pulmonary disease (COPD); painful and inflamed joints muscles and tendons; lupus; inflammatory bowel disease (IBD), including Crohn's disease and ulcerative colitis; giant cell arteritis and polymyalgia rheumatic; and multiple sclerosis (MS), among others. Corticosteroids can also be used to replace certain hormones that are not being produced by the body naturally - for example, in people with Addison's disease.

Dexamethasone and its derivatives are almost pure glucocorticoids, while prednisone and its derivatives have some mineralocorticoid action in addition to the glucocorticoid effect. Fludrocortisone (Florinef) is a synthetic mineralocorticoid. Hydrocortisone (cortisol) is typically used for replacement therapy, e.g. for adrenal insufficiency and congenital adrenal hyperplasia. Corticosteroid is a treatment for Mycosis Fungoides.

Corticosteroids are available in different forms, including: tablets (oral steroids), injections (which can be into blood vessels, joints or muscles), inhalers (such as mouth or nasal sprays), lotions, gels or creams (topical steroids).

Although the use of corticosteroids has been commonplace for several years, they are not always effective and significant side effects do occur, despite not inducing an adequate therapeutic response.

In view of the high number of possible side-effects, corticosteroids are only prescribed if the potential benefits of treatment outweigh the risks and, if prescribed, it is done at the lowest effective dose for the shortest possible time.

On the other hand, glucocorticosteroids (GCs) remain the first-line of treatment for moderate-to-severe active ulcerative colitis (UC). However, up to 40% of patients do not have a suitable response and require rescue therapies such as calcineurinics, anti-TNFs or even colectomy (Liaó J. et al., "Improved outcome of acute severe ulcerative colitis while using early predictors of corticosteroid failure and rescue therapies", Dig Liver Dis. 2016 Jun;48(6):608-12). Using clinical and biological parameters (mainly, the number of motions/day, rectal bleeding, and C-reactive protein levels), steroid-refractoriness can be predicted after 3 to 5 days of GCs therapy. Unfortunately, to date it is not possible to identify those patients who will not respond to GCs before starting therapy.

Naves J.E. et al., "DOP013 Pre-treatment differential microRNA expression profile in ulcerative colitis patients according to their response to corticosteroids" Journal of Crohn's and Colitis, vol. 8, discloses the miRNA expression profile in rectal mucosa of UC patients responding and non-responding to corticosteroids. This document is silent about miR218-5p or its use as a biomarker.

WO2012174293 discloses a method for identifying an inflammatory bowel disease (IBD) patient with dysplasia or cancer based on a miRNA expression profile. This document is silent about miR218-5p or its use as a biomarker. Therefore, there is the need to provide a tool to determine whether a particular subject can positively respond to a corticosteroid treatment or not prior starting its administration.

### SUMMARY OF THE INVENTION

The invention is set out in the appended claims.

The present inventors, working with a group of subjects suffering from IBD, in particular from UC, have found that when miR218-5p was detected in an isolated sample from a subject, it was a reliable predictor of the response of that subject to a corticosteroid-based therapy.

In particular, the inventors surprisingly found in plasma samples taken prior to the start of the administration of the corticosteroid therapy that miR-218-5p was absent in subjects that were later confirmed as therapy responders; whereas a substantial amount of this marker was found in subjects later confirmed as non-responders' samples. That is, miR218-5p is detected in the test sample when the subject is non-responder to the corticosteroid therapy (see Table 2 below).

And, what was even more remarkably, was the fact that miR218-5p allowed classifying glucocorticoid responders and non-responders with 100% of sensitivity, as shown in Table 1. This means that this marker does not provide false positives in the differentiation between responders and non-responders.

The data provided in the present application, therefore, means a great advance in corticosteroid management because using non-invasive techniques, such as a sample of plasma, the physician can easily determine, with high accuracy, whether a subject potentially in need of a corticosteroid therapy, will positively respond, just checking the presence of miR-218-5p. With this quick test, the physician would be able to optimize the therapy and readjust it depending on miR218-5p levels.

Finally, it is also surprising that miR218-5p is able to provide information about corticosteroid therapy efficacy in view of state of the art where it had been reported this marker, up to now, as being related to cancer, in particular to metastatic colorectal cancer, glioblastoma cancer, mesenchymal glioblastoma multiforme, brain cancer, or medulloblastoma, among others.

Thus, in one aspect the present invention provides a method for predicting subject's responsiveness to a glucocorticosteroid medical regimen, the method comprising the step of determining the amount of miR218-5p in a test sample of the subject, isolated prior to starting the medical regimen, wherein (a) if no miR218-5p amount is present in the sample, it is indicative that the subject will be responder to the medical regimen, and (b) if miR218-5p is detected in the test sample it is indicative that the subject will not respond to the medical regimen, and wherein the subject suffers from ulcerative colitis; the isolated test sample is selected from: plasma, serum, and blood, particularly plasma.

In a second aspect the present invention provides the use of primers of probers specific for miR218-5p for predicting subject's responsiveness to a glucocorticosteroid medical regimen prior to the start of said medical regimen, wherein the subject suffers from ulcerative colitis.

In a third aspect the present invention provides the use of miR218-5p alone or in combination with one or more of miR6754-5p, miR4767 and CRP as marker(s) for predicting subject's responsiveness to a glucocorticosteroid medical regimen prior to the start of the medical regimen, wherein the subject suffers from ulcerative colitis.

The present inventors have also surprisingly found that determining the level of miRNA218-5p, miRNA6754 and miR4767, it is also possible to accurately differentiate a subject suffering from UC, from a healthy subject.

In particular, as it is shown below, this miRNA combination was 20-fold decrease in UC subjects, when compared to healthy subjects. This remarkably difference is an evidence of the specificity and selectivity of the combination.

### DETAILED DESCRIPTION OF THE INVENTION

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

The present invention provides, in a first aspect, a method for predicting subject's responsiveness to a glucocorticosteroid medical regimen, the method comprising the step of determining the amount of miR218-5p in an test sample of the subject isolated prior to starting the medical regimen, wherein (a) if no miR218-5p amount is present in the sample, it is indicative that the subject will be responder to the medical regimen, and (b) if miR218-5p is detected in the test sample it is indicative that the subject will not respond to the medical regimen, and wherein the subject suffers from ulcerative colitis; the isolated test sample is selected from: plasma, serum, and blood, particularly plasma.

In the present invention, the term "corticosteroid medical regimen" refers to a medical treatment comprising the administration of one or more corticosteroid drugs.

Described herein but not forming part of the invention is a method is for predicting subject's responsiveness to a corticosteroid drug.

In the present invention miR-218-5p, also known in the state of the art as "hsa-miR218-5p" or "hsa-miR-218", has the sequence SEQ ID NO: 1: UUGUGCUUGAUCUAACCAUGU. Little is known about miR-218-5p. It has been related to cancer development through stem cell regulation. miR-218 precursor family has been reported to be key players in motor neurons development. miR-218-5p shows 794 validated targets (MirTarBase [http://mirtarbase.mbc.nctu.edu.tw]), although only 19 have been validated using a highly reliable technique: BMI1, CDH2, CDK6, DKK2, GLI2, HMGB1, HOXB3, IKBKB, LAMBS, LEF1, MITF, OTUD7B, PDGFRA, RET, RUNX2, SFRP2, SOST, SP1 and TFF1.

As it has been stated above, miR218-5p allows the differentiation of a non-responder from a responder to the corticosteroid medical regimen. Described herein but not forming part of the invention is a method which comprises diagnosing a non-responder from a responder to the corticosteroid drug.

As it is illustrated below, the samples from responder subjects do not have the biomarker, whereas the non-responder does it. Thus, a qualitative criterion can be established on the basis of the information provided by miR218-5p: if no miR218-5p is detected in the test sample, it is indicative that the subject is responder, whereas if miR218-5p is detected in the test sample, at any amount, it can be indicative that the subject is non-responder.

Alternatively, a quantitative criterion can also be established for predicting the subject's responsiveness to the medical regimen by comparing the determined amount with a reference value. Thus, in another embodiment of the first aspect of the invention, the method comprises: (a) determining the amount of miR218-5p; and (b) comparing the amount obtained in (c) with a reference value.

In the present invention, the term "reference value" referred herein is to be understood as a predefined value of a given molecular marker, in the present case any of the biomarkers of the invention as well as in particular embodiments, which is derived from the levels of said molecular marker in a sample or group of samples. The samples are taken from a subject or group of subjects wherein the presence, absence, stage, or course of the disease has been properly performed previously. In the present case, the subject(s) can be subjects who have been already diagnosed as corticosteroid respondents. This value can be used as a threshold to discriminate responders from non-responders. Methods for obtaining the reference value from the group of subjects selected are well-known in the state of the art (Burtis C. A. et al., 2008, Chapter 14, section "Statistical Treatment of Reference Values"). As the skilled person will appreciate, optimal cut-off value will be defined according to the particular applications of the diagnostic or prognostic method: purpose, target population for the diagnosis or prognosis, balance between specificity and sensibility, etc.

In another embodiment of the method of the first aspect of the invention, the method further comprises determining the amount of at least one of miR-6754-5P, miR-4767, C-reactive protein (CRP), or a combination thereof.
miR-6754-5p, also known in the state of the art as "hsa-miR6754-5p", has sequence SEQ ID NO: 2: CCAGGGAGGCUGGUUUGGAGGA
miR-4767, also known in the state of the art as "hsa-miR4767", has the sequence SEQ ID NO: 3: CGCGGGCGCUCCUGGCCGCCGCC.

The amount of these miRNAs referred in the first aspect of the invention or in any of the embodiments of the first aspect of the invention, can be determined by routine methods such as quantitative PCR. Alternatively, the determination of the amount of miRNAs can be performed: (i) extracting miRNA content of the isolated test sample; (ii) sequencing miRNA(s), and (iii) determining the number of reads using an alignment algorithm.

C-reactive protein is an annular, pentameric protein found in blood plasma, whose levels rise in response to inflammation. CRP is used mainly as a marker of inflammation. ELISA, immunoturbidimetry, nephelometry, rapid immunodiffusion, and visual agglutination are all methods used to measure CRP, and kits for its determination are commercially available.

As it is illustrated below, when the expression level of miR-218-5p, miR-6754-5P and miR-4767 before glucocorticoids was determined in samples from responder and non-responders groups, it was found that the resulting score, show a decrease about 20-fold in case of non-responders vs. responders. That is, these particular combination of miRNAs have a well-differentiated profile when both populations are compared, which is indicative of the high sensitivity and accuracy in the differentiation.

When any of the methods of the invention comprises measuring the level of more than one biomarker, the experimental data thus obtained is submitted to statistical methods or algorithms, such as Bowtie 2 algorithm (Langmead B. et al., 2012; Griffiths-Jones S. et al., 2006) to obtain a single value (score). Alternatively, the experimental data thus obtained is integrated by computational Systems Biology-based methods or algorithms (Herrando-Grabulosa M. et al., "Novel Neuroprotective Multicomponent Therapy for Amyotrophic Lateral Sclerosis Designed by Networked Systems", PLoS One 2016, vol. 11:e0147626; Gómez-Serrano M. et al., "Proteome-wide alterations on adipose tissue from obese patients as age-, diabetes- and gender-specific hallmarks", Sci. Rep., 2016, vol. 6:25756) The score thus obtained from the test sample will be compared with the score obtained from control samples (reference value) (see table 2 and 3).

In view of the teachings provided herein, the skilled person in the art using the general knowledge can develop other algorithms to achieve the score from the information provided by the combination of biomarkers. The particular algorithm used in obtaining the score does not limit, at any extend, the usability of the biomarkers of the invention.

In one embodiment of the first aspect of the invention, optionally in combination with any of the embodiments provided above or below, the method further comprises determining the amount of miR-6754-5p and miR-4767.

In another embodiment of the first aspect of the invention, optionally in combination with any of the embodiments provided above or below, the method further comprises determining the amount of miR6754-5p, miR-4767, and CRP.

As it is also illustrated below, when miR-218-5p and CRP were determined, the resulting score provides useful information for significantly differentiate between responders and non-responders.

Thus, in another embodiment of the first aspect of the invention, optionally in combination with any of the embodiments provided above or below, the method comprises the determination of the amount of miR218-5p and CRP in the test sample. In another embodiment of the first aspect of the invention, optionally in combination with any of the embodiments provided above or below, the method comprises: (a) determination of the amount of miR218-5p; (b) determination of CRP concentration; (c) determining a score miR218-5-p/CRP; and (c) compared the score from step (c) with a reference value. In this embodiment, when the reference value is obtained from a subject or group of subjects which have been already identified as corticosteroid responders, if the ratio value is above the reference value, it will be indicative that the subject will be non-responder.

In the present invention, the "glucocorticoid medical regimen" means that the medical regimen comprises one or more glucocorticoids.

Illustrative non-limitative examples of glucocorticoids are cortisol (hydrocortisone), cortisone, prednisone, prednisolone, methylprednisolone, dexamethasone, betamethasone, triamcinolone, fludrocortisone acetate, and deoxycorticosterone acetate, among others. In one embodiment, the glucocorticoid therapy is prednisone or prednisolone.

In another embodiment of the first aspect of the invention, optionally in combination with any of the embodiments provided above or below, the medical regimen is for oral or intravenous therapy.

In another embodiment of the first aspect of the invention, optionally in combination with any of the embodiments provided above or below, the isolated test sample is plasma.

In another embodiment of the first aspect of the invention, optionally in combination with any of the embodiments provided above or below, the method is for predicting subject's responsiveness to a glucocorticosteroid medical regimen, the method comprising the step of determining the amount of miR218-5p in a test sample of the subject, isolated prior to starting the medical regimen; wherein the detection of miR218-5p is indicative that the subject is non-responder to the glucocorticosteroid drug.

In another embodiment of the first aspect of the invention, optionally in combination with any of the embodiments provided above or below, the method is for predicting subject's responsiveness to a glucocorticosteroid medical regimen, the method comprising the step of determining the amount of miR218-5p in a test sample of the subject, isolated prior to starting the medical regimen; wherein the determination of the amount of miRNA218-5p is performed: (i) extracting miRNA content of the isolated test sample; (ii) sequencing miRNA(s), and (iii) determining the number of reads using an alignment and wherein the detection level of miR218-5p is indicative that the subject is non-responder to the glucocorticosteroid drug.

In another embodiment of the first aspect of the invention, optionally in combination with any of the embodiments provided above or below, the method is for predicting subject's responsiveness to a glucocorticosteroid medical regimen, the method comprising the step of determining the amount of miR218-5p in a test sample of the subject, isolated prior to starting the medical regimen; wherein the determination of the amount of miRNA218-5p is performed: (i) extracting miRNA content of the isolated test sample; and (ii) quantifying the microRNA by PCR (such as Real-time PCR).

In another embodiment of the first aspect of the invention, optionally in combination with any of the embodiments provided above or below, the method is for predicting subject's responsiveness to a glucocorticosteroid medical regimen, the method comprising the step of determining the amount of miR218-5p, miR6754-5p, and miR4767, in a test sample of the subject, isolated prior to starting the medical regimen; wherein the determination of the amount of miRNAs is performed: (i) extracting miRNA content of the isolated test sample; and (ii) quantifying the amount of microRNAs.

In another embodiment of the first aspect of the invention, optionally in combination with any of the embodiments provided above or below, the method is for predicting subject's responsiveness to a glucocorticosteroid medical regimen, the method comprising the step of determining the amount of miR218-5p, miR6754-5p, and miR4767, in a test sample of the subject, isolated prior to starting the medical regimen; wherein the determination of the amount of miRNAs is performed: (i) extracting miRNA content of the isolated test sample; (ii) quantifying the microRNA by PCR (such as Real-time PCR).

As mentioned above, when more than one mRNA is used in any of the methods of the invention, a statistical method or algorithm is used to get a single score starting from the measures of the different biomarkers.

In another embodiment of the first aspect of the invention, optionally in combination with any of the embodiments provided above or below, the method is for predicting subject's responsiveness to a glucocorticosteroid medical regimen, the method comprising the step of determining the amount of miR218-5p and CRP in a test sample of the subject, isolated prior to starting the medical regimen; wherein (a) it is determined of the amount of miR218-5p; (b) it is determined CRP concentration; (c) it is determined the score between miR218-5-p and CRP; and (c) it is compared the value from step (c) with a reference value.

In a second aspect, the present invention provides the use of primers or probes for determining the amount of miR218-5p for predicting subject's responsiveness to a glucocorticosteroid medical regimen prior to the start of said medical regimen, wherein the subject suffers from ulcerative colitis.

In an embodiment of the second aspect of the invention, the use further comprises means for determining the amount of one or more of miR6754-5p, miR4767, and C-reactive protein. In another embodiment of the second aspect of the invention, the means are for determining the amount of miR218-5p, miR6754-5P, and miR4767. In another embodiment of the second aspect of the invention, the means are for determining the amount of miR218-5p and CRP.

In another embodiment of the second aspect of the invention, the specific primers or probes are optionally labelled.

Both primers and probes can be designed from the particular miRNA sequence using routine bioinformatics tools.

In a third aspect the present invention provides the use of miR218-5p alone or in combination with one or more of miR6754-5p, miR4767 and CRP, as a marker for predicting subject's responsiveness to a glucocorticosteroid medical regimen prior to the start of the medical regimen, wherein the subject suffers from ulcerative colitis.

All the embodiments provided above for the first aspect of the invention, are also embodiments of the third aspect of the invention.

The term "diagnosis" is known to the person skilled in the art. As used herein "diagnosis" is understood as becoming aware of a particular medical condition complication or risk in a subject; the determination of the nature of the disease or condition; or the distinguishing of one disease or condition from another. It refers both to the process of attempting to determine or identify the possible disease or disorder, and to the opinion reached by this process. A diagnosis, in the sense of diagnostic procedure, can be regarded as an attempt at classification of an individual's condition into separate and distinct categories that allow medical decisions about treatment and prognosis to be made. Subsequently, a diagnostic opinion is often described in terms of a disease or other condition. However, a diagnosis can take many forms. It might be a matter of detecting the presence and naming the disease, lesion, dysfunction or disability. It might be an exercise to attribute a category for management or for prognosis. It may indicate either degree of abnormality on a continuum or kind of abnormality in a classification.

"Prognosis" as used herein refers to the prediction of the probable progression and outcome of a disease.

The term "inflammatory gastrointestinal disease or disorder", referred in any of the aspects provided by the present invention, includes an inflammation gastrointestinal disease or disorder such as Inflammatory Bowel Disease (IBD), Crohn's disease, colitis, enteroinvasive colitis, C. difficile colitis, ulcerative colitis (UC), Inflammatory Bowel Syndrome (IBS), pouchitis, diverticulitis, gastroenteritis, colic, appendicitis, appendicitis, ascending colangitis, esophagitis, gastritis, or enteritis.

The step of determining the amount of miR218-5p, miR6754-5p and miR4767 referred in any of the aspects fourth to seventh can be performed using routine protocols for those skilled in the art, such as quantitative amplification, or real-time PCR. The amount of each one of the biomarkers can be determined in separate tests or at the same time. Details about the detection have already been explained above and a particular embodiment is provided below in the section of "Examples".

And then, as explained above, a single score can be determined starting from the independent measures, using algorithms as those provided above.

Means to quantify miR218-5p, miR6754-5p and miR4767 are specific primers or probes.

The means used for determining the amount of the biomarkers can form part of a kit.

The concentration of C reactive protein can be determined using any of the commercially available kits. Once the concentration of the C reactive protein is determined, this value can be statistically treated together with the total amount of miR218-5p to achieve one single value. Algorithms to appropriately manage these data and obtain one single value are also available in the state of the art. Similar to the miRNAs combination, an algorithm can be used to obtain a single value (score) starting from the measure of each one of the biomarkers separately.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### EXAMPLES

### 1. Materials and Methods

### 1.1. Patients

Twenty-four patients with moderately-to-severe active UC and 10 healthy controls were included. Clinical disease activity was assessed according to the Montreal criteria (Silverberg M. S. et al., 2005).

Healthy controls were identified from the Endoscopy Unit, and they were invited to participate in case of no familiar or personal history of IBD was present and no pathologic findings were observed in a complete colonoscopy.

Written informed consent was obtained from all the patients and controls.

All UC patients were treated with 0.75-1 mg/kg/day of oral prednisone for moderate flares or 1mg/kg/day of intravenous prednisolone in case of severe activity. After 3 to 5 days of GC therapy, patients started rescue therapy with cyclosporin or infliximab if they still presented more than 8 stools/day, or 4 to 8 stools/day together with a C-reactive protein (CRP) > 45 mg/L measured by automated immunoassay using an ultrasensitive CRP test (N High Sensitivity CRP) on a BN-ProSpec nephelometer (Dade Behring, GMBH, Marburg, Germany). At day 7 from baseline, patients were assessed clinically and grouped as steroid-responsive (if they have mild activity or inactive disease according to the severity criteria of the Montreal classification) or steroid-refractory (if moderate or severe clinical activity was still present, or they started rescue therapy).

### 1.2. Small RNA extraction from plasma samples

Small RNAs (<1000nt) were isolated from 300µl of plasma using miRCURY RNA insolation kit (EXIQON, Cat. No.300112) following the manufacturer's instructions. Then, RNA quality of isolated microRNA (miRNA) were assessed with miRCURY^{™} microRNA QC PCR kit (EXIQON, Cat. No.203848).

### 1.3. Plasmatic miRNAs sequencing

Circulating small RNA sequencing was carried out using TruSeq Small RNA Sample Prep Kit (Illumina, USA) with some modifications. The selection of the library (135-160bp) was performed automatically with PippinPrep 3% agarose cassettes (BluePippin^{™} cassette kit, Sage Science Inc., USA) in the Bioanalyzer (Agilent) by means of PippinPrep software v.6.0. In addition, QIAquick column purifications were performed prior and after to size selection, and Kappa Library Quantification Kit was also used to PCR quantification. Sequencing was developed on HiSeq2500 platform (Illumina Inc.) using adapted protocols for TruSeq SR cluster kit v3 (Part#15023335) and HiSeq GA (Part#15050107), and following guidelines of cBot^{™} (Part#15006165) and HiSeq2500 System (Part#15035786). Raw data was analysed by Real Time Analysis software v. 1.17.21.3 and Sequence Analysis Viewer software v. 1.8.20 (Illumina Inc.).

### 1.4. Data compilation and mapping

### microRNAs:

Quality control of raw data from microRNA sequencing was performed according to Trimmomatic methods to remove the adapter sequences from the reads (Bolger A. M. et al., 2014). The data for each sample was aligned to the miRNA sequences using Bowtie 2 algorithm (Langmead B. et al., 2012; Griffiths-Jones S. et al., 2006). For each sample, the number of reads that map to a particular miRNA sequence was counted (Langmead B. et al., 2012; Griffiths-Jones S. et al., 2006). The miRNAs for which there was no enough signal was discarded (if none of the samples has 10 counts of the miRNA and/or none of the samples has 2 counts per million). The count matrix was normalized through the weighted trimmed mean of M-values (Robinson M. D. et al., 2010). Then, the differential expression was calculated through edgeR and limma's voom methods (Robinson M. D. et al., 2010; Ritchie M. E. et al., 2015). Those miRNAs complying with the following criteria were considered differentially expressed and functionally relevant: Adjusted p-value (FDR) < 0.05, or classifier p-value < 0.05, or non-adjusted p-value < 0.01; and Log FC > 0.58 (i.e. the counts in one cohort is at least 50% higher than in the other).

### 1.5. Biomarker identification

The expression sample by sample of each miRNA measured by sequencing as disclosed in section 1.3. above was used to identify the miRNAs that better classify the samples in responder and non-responders UC patients.

In order to identify the best combinations of miRNAs that classified the samples in responder and non-responders, the approach disclosed by Kira et al., 1992 were used to identify potential sets of biomarkers from miRNA data. These calculations were performed with all the miRNAs sequenced or with those miRNAs more related to the MoA. This protocol was performed in basal time and day 3 of treatment in plasma samples to identify potential biomarkers at both times. The following parameters were used in the selection of the top-ranked miRNAs:
- Accuracy: Proportion of samples correctly classified.
- Generalization Capability: probability to classify the set of samples of two cohorts of high-throughput by using Leave-One-Out strategy, it means, probability to predict correctly a sample that never before have been analysed. It depends strictly on the set of data used for the analysis, and thus, it is function of the quantity and quality of the data set.
- Sensitivity: proportion of positives that are correctly identified as such. [Sensitivity = True Positives/Positives]
- Specificity: proportion of negatives that are correctly identified as such. [Specificity= True Negatives/Negatives]
- Precision: proportion of samples classified as positives that are true positives. [Precision= True Positives/Positive calls]
- Negative predictive value (NPV): proportion of samples classified as negatives that are true negatives. [NPV= True Negatives/Negative calls]

### 1.6. Quantification of C-reactive protein concentration in plasma samples

Plasma high sensitivity C-reactive protein (CRP) were measured using an ultrasensitive CRP test (N High Sensitivity CRP) on a BN-ProSpec nephelometer (Dade Behring, GMBH, Marburg, Germany) with an inter-assay variation coefficient of 3.7 and 3.5 % for CRP concentrations of 2.38 and 52.2 mg/l, respectively. Assay was performed in triplicate using diluted samples.

### 1.7. Combinational algorithms

The predictive algorithm is formed by ensembles of 3 (PCR+miR-218-5P) or 4 (3 miRNAs) shallow artificial neural networks (multilayer perceptrons with one hidden layer with two nodes), obtained from the evaluation of the classification power of each feature as presented in the experimental data of responder and non-responder patients
i) Algorithm for combination of 3 miRNAs:

| | |
|---|---|
| A | (((1/(1+EXP(MIN(-2*((((2/(1+EXP(MIN(-2*(654,4567331+(((((**X-**0,306487)/4,3913249)*2)-1)*736,3770017)+(((((**Y-**-0,3140988)/4,3716006)*2)-1)*-1171,6592461)+(((((**Z**--0,3653696)/4,466369)*2)-1)*1133,1964147));709))))-1)*-92,412192)+91,3133945);709))))*2)+-1) |
| B | (((1/(1+EXP(MIN(-2*((((2/(1+EXP(MIN(-2*(21,6875279+((((( **X-**0,306487)/4,3913249)*2)-1)*24,7138138)+((((( **Y** --0,3140988)/4,3716006)*2)-1)*-40,1160305)+((((( **Z -**-0,3653696)/4,466369)*2)-1)*38,7102354));709))))-1)*-63,5425749)+0,1477523);709))))*2)+-1) |
| C | (((1/(1+EXP(MIN(-2*((((2/(1+EXP(MIN(-2*(-21,6206271+((((( **X-**0,306487)/4,3913249)*2)-1)*-24,5814867)+((((( **Y** --0,3140988)/4,3716006)*2)-1)*39,8176042)+((((( **Z** --0,3653696)/4,466369)*2)-1)*-38,4698272));709))))-1)*64,2414559)+-0,3838272);709))))*2)+-1) |
| D | (((1/(1+EXP(MIN(-2*((((2/(1+EXP(MIN(-2*(-931,0908707+((((( **X-**0,306487)/4,3913249)*2)-1)*-1038,4949607)+((((( **Y** --0,3140988)/4,3716006)*2)-1)*1530,401108)+((((( **Z** --0,3653696)/4,466369)*2)-1)*-1480,2745616));709))))-1)*111,1111977)+110,0125712);709))))*2)+-1) |
| **TOTAL** | **(((A+B+C+D)/4)+1)/2** |

| | |
|---|---|
| **X=** ([hsa-miR-218-5p value] - 3,14254134879075E-07)/ 1,02534231780687E-06 **Y**= ([hsa-miR-6754-5p value ] - 3,61761554739333E-07)/ 1,15174433187936E-06 **Z=** ([hsa-miR-4767 value] - 1,49655376842361E-06)/ 4,09599941391501E-06 | |

ii) Algorithm for combination of protein C reactive and hsa-miR-218-5p

| | |
|---|---|
| A | (((1/(1+EXP(-2*((((2/(1+EXP(-2*(2,1408286+(((((**X**-0)/46,8581222)*2)-1)*11,6020691)+(((((**Y**-0)/1)*2)-1)*4,1641155)+(((((**Z**-0)/1)*2)-1)*-4,8870598)))))-1)*-10,484203)+(((2/(1+EXP(-2*(-1,1555452+((((( **X** -0)/46,8581222)*2)-1)*0,1506293)+((((( **Y** -0)/1)*2)-1)*-1,9251207)+((((( **Z** -0)/1)*2)-1)*2,8786841)))))-1)*-7,6593626)+-7,5406107))))*2)+-1) |
| B | (((1/(1+EXP(-2*((((2/(1+EXP(-2*(-2,8472913+((((( **X** -0)/46,8581222)*2)-1)*1,6714154)+((((( **Y** -0)/1)*2)-1)*-0,2212944)+((((( **Z** -0)/1)*2)-1)*2,3494935)))))-1)*-0,774982)+(((2/(1+EXP(-2*(9,2883611+((((( **X** -0)/46,8581222)*2)-1)*9,2706309)+((((( **Y** -0)/1)*2)-1)*0,0019549)+((((( **Z** -0)/1)*2)-1)*0,3698876)))))-1)*-14,3022544)+-1,1813372))))*2)+-1) |
| C | (((1/(1+EXP(-2*((((2/(1+EXP(-2*(-0,7138477+((((( **X** -0)/46,8581222)*2)-1)*-0,0422662)+((((( **Y** -0)/1)*2)-1)*-2,1430485)+((((( **Z** -0)/1)*2)-1)*1,9640948)))))-1)*-7,5699713)+(((2/(1+EXP(-2*(-2,1761004+((((( **X** -0)/46,8581222)*2)-1)*-11,5279685)+((((( **Y** -0)/1)*2)-1)*-3,3761391)+((((( **Z** -0)/1)*2)-1)*5,5690494)))))-1)*10,3434871)+-7,426751))))*2)+-1) |
| **TOTAL** | **(A + B + C)/3** |

| | |
|---|---|
| **X=** [hsa-miR-218-5p value]/0,0961 **Y**= [BASAL_PCR < 40, value] **Z=** [BASAL_PCR > 40, value ] | |

### 1.7. Quantification of mRNAs using quantitative real-time PCR

Total RNA (mRNA and miRNA) is extracted from plasma samples using the commercial kit based on retention columns miRCURY RNA Isolation_Biofluids (Exiqon, USA), following the manufacturer's recommendations. Lysis buffer and isopropanol is used to precipitate the proteins from 200 µl of non-haemolysed plasma. After centrifugation, the supernatants are transferred to retention columns, and the RNA is recovered with PCR grade water. Total RNA integrity is assessed in TapeStation system (Agilent Technologies, Spain).

MiRNA assessment by real time-PCR is done in Pick-&-Mix microRNA PCR panels (Exiqon), including pre-designed plaques with the selected miRNAs primers. The Pick-&-Mix panels are developed by means of LNA^{™} (Locked Nucleic Acids^{™}, Exiqon) oligonucleotide methodology.

Total RNA is retrotranscribed (RT) to cDNA using the Universal cDNA synthesis kit (Exiqon). Twenty ng of the resulting cDNA are added to the SYBR^{©} Green Master Mix (Exiqon) solution in a final volume of 10µl in each well. Each sample is evaluated in triplicate by means of fluorescence signals with LightCycler 480 (Roche, USA) thermocycler. Synthetic miRNAs (Spike-ins, Exiqon) and 6 small RNA/microRNA reference genes (U6, SNORD38B, SNORD49A, miR-103-3p, miR-191-5p and hsa-miR-423-5p) are used as internal control or as housekeeping gene, respectively (https://www.exiqon.com/mirna-pcr). Data normalization and quantification is developed with the Exiqon GenEx qPCR analysis software (http://www.exiqon.com/qpcr-software). The values are expressed as relative quantities or fold changes, and they will are obtained from PCR efficiencies and crossing point deviation of a sample and the reference control (Pfaffl M. W. Nucleic Acid Res 2001, 29(9):e45).

### 2. RESULTS

### Patients and controls

Of the 24 UC patients included in the study, thirteen were classified as responder and 11 as non-responder to 7 days after starting GC treatment. When comparing the main epidemiological and clinical variables, statistically significant differences associated with the severity of the flare-up (Montreal basal rate and basal PCR) were found between responders and non-responders.

### Identification of circulating microRNAs predictors of GC response

The miRNA transcriptome from the blood was statistically analysed by Systems Biology methods as described above in order to identify potential miRNA biomarkers. The best miRNA combinations were selected by higher accuracy, higher generalization capability and lower number of miRNAs.

The best microRNA or combination thereof was obtained through the analysis of the whole pool of miRNAs. Thus, the miR-218-5p showed the best values as classifier of UC patients according to GC response (Accuracy=84.21%, Generalization Capability=84.21%, Sensitivity=100%, Specificity=66.67%, Precision=76.92%, NPV=100%) (Table 1).

In addition, this same miRNA was included in the highest classifier combination, together with the miR-6754-5p and miR-4767 (Accuracy=94.74%, Generalization Capability=75.44%, Sensitivity=70%, Specificity=81.48%, Precision=81.45%, NPV=70.77%).

This miRNA combination provides better accuracy, specificity and precision than the miR-218-5p alone:

**Table 1**

| **mirna** | | | | | |
|---|---|---|---|---|---|
| | ***Accuracy (SEM)*** % | **Sensitivity (SEM)** % | **Specificity (SEM)** % | ***Precision* (SEM)** % | ***NPV* (SEM) %** |
| **MIR-218-5P** | 84,21 (0,00) | 100,00 (0,00) | 66,67 (0,00) | 76,92 (0,00) | 100,00 (0,00) |
| **MIR-4767** | 68,42 (0,00) | 0,00 (0,00) | 66,67 (0,00) | 0,00 (0,00) | 37,50 (0,00) |
| **MIR-6754-5P** | 68,42 (0,00) | 30,00 (0,00) | 88,89 (0,00) | 75,00 (0,00) | 53,33 (0,00) |
| **MIR-218-5P +MIR-6754-5P** | 84,21 (0,00) | 100,00 (0,00) | 66,67 (0,00) | 76,92 (0,00) | 100,00 (0,00) |
| **MIR-218-5P +MIR-4767** | 84,21 (0,00) | 100,00 (0,00) | 66,67 (0,00) | 76,92 (0,00) | 100,00 (0,00) |
| **MIR-6754-5P + MIR-4 767** | 74,85 (1,77) | 30,00 (0,00) | 75,31 (1,63) | 57,78 (1,47) | 49,15 (0,57) |
| **MIR-218-5P +MIR-6754-5P+MIR-4767** | 94,74 (1,52) | 70,00 (0,00) | 81,48 (3,21) | 81,45 (2,56) | 70,77 (0,87) |

Table 2 shows the mean and the interquartile range of selected microRNAs values. These expression values were statistical compared using the non-parametric U-Mann Whitney test for independent samples (patients *versus* control, steroid responsive *versus* steroid-refractary, steroid responsive *versus* control, steroid-refractary *versus* control), and the Wilcoxson test for comparisons at baseline *versus* after three days of GC starting. The results showed higher levels of miR-218-5p in steroid-refractory than in the steroid-sensitive UC patients at baseline (p=0.013). The scores resulting of the miR-218-5p, miR-6754-5p and miR-4767 combination at baseline perfectly classified both groups of patients with different GC response (p=0.000), as well as steroid-refractory patients and controls (p=0.031). hsa-miR-218-5P, hsa-miR-6754-5p and hsa-miR-4767 in combination, therefore, have great predictive ability of the steroid-refractoriness in UC.

**Table 2**

| | Baseline | | | | 3rd day of treatment | | | |
|---|---|---|---|---|---|---|---|---|
| | ***hsa-miR-218-5p*** (counts ×10-7) | ***hsa-miR-6754-5p*** (counts ×10-7) | ***hsa-miR-4767*** (counts ×10-7) | ***Combi3miRs** (Relative units)* | ***hsa-miR-218-5p*** (counts ×10-7) | ***hsa-miR-6754-5p*** (counts ×10-7) | ***hsa-miR-4767*** (counts ×10-7) | *Willcoxson test* |
| **Controls** | 0 (0-0) | 0(0-0) | 0(0-0,2) | 1.00 (0.05-1.00) | - | - | - | **p<0.007 vs.baseline* |
| **All patients** | 0 (0-1,6) | 0 (0-0,5) | 2.9 (0.02-5,6)^{b} | 0.05 (0.05-1.00) | N/A* | N/A* | 1,4 (0-5,1) | |
| **Steroid-responsive patients** | 0 (0-0) | 0 (0-3,8) | 0.14 (0-3.1) | 1.00 (1.00-1.00) | N/A* | N/A* | 0 (0-3,9) | |
| **Steroid-refractory patients** | 1,6 (0-2,7)^{a} | 0 (0-0) | 5,6 (2,8-8,0)^{b} | 0.05 (0.05-0.05)^{c} | N/A* | N/A* | 3,4 (0-6,5) | |
| *U-Mann Whitney test* | *^{a}p=0,013 vs. steroid-responsive; ^{b}p<0,05 vs. controls; ^{c}p≤0.031 vs. steroid-responsive and controls* | | | | | | | |

### The microRNA combination allows the differentiation between healthy subjects and UC subjects

Among the data obtained, it was found that the combination of miRNAs miR218-5p, miR6754-5p and miR4767 was differentially expressed in healthy and UC subjects.

In particular, it was found that in healthy subjects the combination was expressed, in relative units, at 1.00, whereas in UC patients the combination was expressed, in relative units, at 0.05. That is, the combination was 20-fold under-expressed in UC subjects when compared with healthy subjects.

These allow to conclude that this miRNA combination can be used in the efficient differentiation between healthy and UC subjects.

### The combination of miR218-5p and C-reactive protein allows the efficient differentiation between responders and non-responders

As it is shown in Table 3 below, when the miR218-5p expression was used in combination with the C-reactive protein concentration it was found that it could be used as accurate criterion for classifying the subject as responder/non-responders. In particular, it was found that the score value combining the miR-128 and CRP was substantially increased respect to controls, in steroid-responder patients whereas it was substantially reduced steroid-refractory patients:

**Table 3**

| | **Baseline** |
|---|---|
| | ***hsa-miR-218-5p*/*CRP*** (score) |
| **Steroid-responsive patients** | 09995767 (0.9995767-09995767) |
| **Steroid-refractory patients** | -0.999875 (-0.999875-0.00149)* |
| *U-Mann Whitney test: *p≤0.012 vs. steroid-responsive and controls* | |

This means that miRNA combinations comprising CRP correlate extremely well with corticosteroid response in this hospital cohort.

### REFERENCES CITED IN THE APPLICATION

Burtis C. A. et al., 2008, Chapter 14, section "Statistical Treatment of Reference Values"
Silverberg M. S. et al., "Toward an integrated clinical, molecular and serological classification of inflammatory bowel disease: report of a Working Party of the 2005 Montreal World Congress of Gastroenterology", 2005, Can J Gastroenterol., vol. 19(Suppl A), pages 5A-36A.
Shi W. et al., "Optimizing the noise versus bias trade-off for Illumina whole genome expression BeadChips", 2010, Nucleic Acids Res, vol. 38(22):e204.
Bolger A. M. et al., "Trimmomatic: a flexible trimmer for Illumina sequence data", 2014, Bioinformatics, vol. 30(15), pages 2114-20.
Langmead B. et al., "Fast gapped-read alignment with Bowtie 2", 2012, Nat Methods, vol. 9(4), pages 357-9.
Griffiths-Jones S. et al., "miRBase: microRNA sequences, targets and gene nomenclature", 2006, Nucleic Acids Res, vol. 34, D140-4.
Robinson M. D. et al., "A scaling normalization method for differential expression analysis of RNA-seq data", 2010, Genome Biol., vol. 11(3), R25.
Pfaffl MW. A new mathematical model for relative quantification in real-time RT-PCR. Nucleic Acids Res 2001; 29(9):e45.
Ritchie M. E. et al., "Limma powers differential expression analyses for RNA-sequencing and microarray studies", 2015, Nucleic Acids Res, vol. 43(7), e47.
Herrando-Grabulosa M. et al., "Novel Neuroprotective Multicomponent Therapy for Amyotrophic Lateral Sclerosis Designed by Networked Systems", PLoS One 2016, vol. 11:e0147626.
Gómez-Serrano M. et al., "Proteome-wide alterations on adipose tissue from obese patients as age-, diabetes- and gender-specific hallmarks", Sci. Rep., 2016, vol. 6:25756
Naves J.E. et al., "DOP013 Pre-treatment differential microRNA expression profile in ulcerative colitis patients according to their response to corticosteroids" Journal of Chrohn's and Colitis, vol. 8.

## Claims

1. A method for predicting subject's responsiveness to a glucocorticosteroid medical regimen, prior to the start of said medical regimen, the method comprising the step of determining the amount of miR218-5p in a test sample of the subject, isolated prior to starting the medical regimen, wherein:
(a) if no miR218-5p amount is present in the sample, it is indicative that the subject will be responder to the medical regimen, and
(b) if miR218-5p is detected in the test sample it is indicative that the subject will not respond to the medical regimen, and
wherein
- the subject suffers from ulcerative colitis;
- the isolated test sample is selected from: plasma, serum, and blood, particularly plasma;

2. The method according to claim 1, which further comprises the step of determining the amount of one or more of the following markers: miR6754-5P, miR4767, and C-reactive protein (CRP).

3. The method according to claim 2, which comprises the step of determining the amount of miR218-5p, miR6754-5P, and miR4767; or alternatively, the method comprises the step of determining the amount of miR218-5p and CRP.

4. The method according to any one of the claims 1-3, wherein the medical regimen is for oral or intravenous administration.

5. Use of primers or probes specific for miR218-5p, for predicting subject's responsiveness to a glucocorticosteroid medical regimen prior to the start of said medical regimen, wherein the subject suffers from ulcerative colitis.

6. Use of miR218-5p as marker for predicting subject's responsiveness to a glucocorticosteroid medical regimen, prior to the start of the medical regimen, wherein the subject suffers from ulcerative colitis.

7. Use of miR-218-5p according to claim 6, in combination with one or more of miR6754-5p, mi-R4767 and CRP for predicting subject's responsiveness to a glucocorticosteroid medical regimen, prior to the start of the medical regimen, wherein the subject suffers from ulcerative colitis.

8. Use of miR-218-5p according to claim 6, in combination with CRP for predicting subject's responsiveness to a glucocorticosteroid medical regimen, prior to the start of the medical regimen wherein the subject suffers from ulcerative colitis.

## Patentansprüche

1. Ein Verfahren zum Vorhersagen des Ansprechens eines Patienten auf ein medizinisches Glucocorticosteroid-Regime vor dem Beginn des medizinischen Regimes, wobei das Verfahren den Schritt des Bestimmens der Menge an miR218-5p in einer Testprobe des Patienten umfasst, die vor dem Beginn des medizinischen Regimes isoliert wurde, wobei:
(a) wenn keine miR218-5p-Menge in der Probe vorhanden ist, dies ein Hinweis darauf ist, dass der Patient auf das medizinische Regime ansprechen wird, und
(b) wenn miR218-5p in der Testprobe nachgewiesen wird, dies ein Hinweis darauf ist, dass der Patient nicht auf das medizinische Regime ansprechen wird, und
wobei
- der Patient an Colitis ulcerosa leidet;
- die isolierte Testprobe ausgewählt ist aus: Plasma, Serum und Blut, insbesondere Plasma;

2. Das Verfahren nach Anspruch 1, welches ferner den Schritt des Bestimmens der Menge von einem oder mehreren der folgenden Marker umfasst: miR6754-5P, miR4767 und C-reaktivem Protein (CRP).

3. Das Verfahren nach Anspruch 2, welches den Schritt des Bestimmens der Menge an miR218-5p, miR6754-5P und miR4767 umfasst; oder alternativ umfasst das Verfahren den Schritt des Bestimmens der Menge an miR218-5p und CRP.

4. Das Verfahren nach einem der Ansprüche 1 bis 3, wobei das medizinische Regime zur oralen oder intravenösen Verabreichung bestimmt ist.

5. Verwendung von Primern oder Sonden, die für miR218-5p spezifisch sind, zur Vorhersage des Ansprechens eines Patienten auf ein medizinisches Glucocorticosteroid-Regime vor dem Beginn des medizinischen Regimes, wobei der Patient an Colitis ulcerosa leidet.

6. Verwendung von miR218-5p als Marker für die Vorhersage des Ansprechens eines Patienten auf ein medizinisches Glucocorticosteroid-Regime vor dem Beginn des medizinischen Regimes, wobei der Patient an Colitis ulcerosa leidet.

7. Verwendung von miR-218-5p nach Anspruch 6 in Kombination mit einem oder mehreren von miR6754-5p, mi-R4767 und CRP zur Vorhersage des Ansprechens des Patienten auf ein medizinisches Glucocorticosteroid-Regime vor dem Beginn des medizinischen Regimes, wobei der Patient an Colitis ulcerosa leidet.

8. Verwendung von miR-218-5p nach Anspruch 6 in Kombination mit CRP zur Vorhersage des Ansprechens des Patienten auf ein medizinisches Glucocorticosteroid-Regime vor dem Beginn des medizinischen Regimes, wobei der Patient an Colitis ulcerosa leidet.

## Revendications

1. Un procédé pour prédire la réactivité d'un sujet à un régime médical de glucocorticostéroïdes, avant le début dudit régime médical, le procédé comprenant l'étape de déterminer la quantité de miR218-5p dans un échantillon d'essai du sujet, isolé avant de commencer le régime médical, dans lequel :
(a) si aucune quantité de miR218-5p n'est présente dans l'échantillon, cela indique que le sujet répondra au régime médical, et
(b) si miR218-5p est détecté dans l'échantillon d'essai, cela indique que le sujet ne répondra pas au régime médical, et
dans lequel
- le sujet souffre de colite ulcéreuse ;
- l'échantillon d'essai isolé est choisi parmi : du plasma, du sérum et du sang, en particulier du plasma ;

2. Le procédé selon la revendication 1, qui comprend en outre l'étape consistant à déterminer la quantité d'un ou plusieurs des marqueurs suivants : miR6754-5P, miR4767 et protéine C-réactive (CRP).

3. Le procédé selon la revendication 2, qui comprend l'étape consistant à déterminer la quantité de miR218-5p, miR6754-5P et miR4767 ; ou en variante, le procédé comprend l'étape consistant à déterminer la quantité de miR218-5p et CRP.

4. Le procédé selon l'une quelconque des revendications 1 à 3, dans lequel le régime médical est pour l'administration orale ou intraveineuse.

5. Utilisation d'amorces ou de sondes spécifiques de miR218-5p, pour prédire la réactivité d'un sujet à un régime médical de glucocorticostéroïdes avant le début dudit régime médical, dans laquelle le sujet souffre de colite ulcéreuse.

6. Utilisation de miR218-5p comme marqueur pour prédire la réactivité d'un sujet à un régime médical de glucocorticostéroïdes, avant le début du régime médical, dans laquelle le sujet souffre de colite ulcéreuse.

7. Utilisation de miR-218-5p selon la revendication 6, en combinaison avec un ou plusieurs parmi miR6754-5p, mi-R4767 et CRP pour prédire la réactivité du sujet à un régime médical de glucocorticostéroïdes, avant le début du régime médical, dans laquelle le sujet souffre de colite ulcéreuse.

8. Utilisation de miR-218-5p selon la revendication 6, en combinaison avec la CRP pour prédire la réactivité du sujet à un régime médical de glucocorticostéroïdes, avant le début du régime médical dans laquelle le sujet souffre de colite ulcéreuse.
